# EUROPEAN PATENT APPLICATION

(11) **EP 1 743 531 A1**
(43) Date of publication of application: **17.01.2007**
(21) Application number: 05720122.0
(22) Date of filing: 07.03.2005
(51) Int. Cl.: A23L 1/33, A23K 1/16, A23L 1/30, A61K 31/122, A61K 31/202, A61K 31/683, A61K 35/56, A61P 3/02

(54) **AGGREGATES OF EYEBALLS OF CRUSTACEANS, USAGE, FOODS COATAINING THE SAME, AND PRODUCTION PROCESSES**

(30) Priority: 08.03.2004 JP 2004063594
(71) Applicant: NIPPON SUISAN KAISHA, LTD., Tokyo 100-8686 (JP)
(72) Inventor: YOSHITOMI, Bunji, c/o Nippon Suisan Kaisha, Ltd, Hachioji-shi, Tokyo 1920906 (JP)
(74) Representative: HOFFMANN EITLE
(86) International application number: PCT/JP2005/003850
(87) International publication number: WO 2005/084463

(57) **Abstract**

[Problems] The present invention provides a method for efficiently utilizing crustaceans, particularly, krill as resources. More particularly, the present invention provides a method for efficiently recovering and utilizing lipophilic effective substances such as carotenoids, highly-unsaturated fatty acids, and phospholipids which are components thereof.

[Means for Solving the Problems] There are provided an aggregate of eyeballs of a crustacean; use of an aggregate of eyeballs of a crustacean as a supply source of a carotenoid, highly-unsaturated fatty acids and/or phospholipids; a food or a nutritional hearth supplement for furnishing a carotenoid, highly-unsaturated fatty acids/or phospholipids, which contains an aggregate of eyeballs of a crustacean as it is or in a pulverized state; and a process for producing an aggregate of eyeballs of a crustacean containing reducing a water content of the crustacean to facilitate the separation of the eyeballs and separating the eyeballs from a fish body by physical impact, followed by recovering them. The crustacean is preferably krill or mysid.

## Description

### Technical Field

The present invention relates to a composition abundantly containing, for example, carotenoids, highly-unsaturated fatty acids and/or phospholipids, which can be utilized in the fields of food, health food and feed.

### Background Art

Astaxanthin is a member of the carotenoid family and is known to have a characteristic red color. The pigment thereof is used not only as a food colorant, but also as a pigment for fish feed and, further, in recent years, its high antioxidative property is attracted and utilized in a health food and the like. As for matters containing natural astaxanthin, salmon's muscles are well known. Besides, natural astaxanthin is contained in many of other animals and plants, and krill is one of them.

In crustaceans such as krill, carotenoids, highly-unsaturated fatty acids, phospholipids and the like are contained, and utilization thereof have been conventionally aimed for. However, contents thereof in the crustaceans are not always high in comparison with other raw materials.

Carotenoids, highly-unsaturated fatty acids, phospholipids and the like are essential components for humans, animals, fish and shellfish, and those originated from various types of raw materials are used. As for commercial supply sources thereof, those which contain them in high concentrations and are easily purified and the like are preferred.

For example, in the case of the krill, a dried product thereof, namely, krill meal is used as a supply source of extraction of carotenoids. The carotenoids content of fresh krill (water content: about 80%) is from 30 to 40 ppm, but a dried product such as krill meal (water content: about 10%) contains more than 200 ppm of carotenoids. When lipids in the krill meal are extracted by using conventional extraction methods (lipid content is about 10% dry basis), the extracted oil contains about 2000ppm of carotenoids.

On the other hand, natural carotenoids available in the market are mainly obtained from raw materials including yeasts such as *Pfaffia rhodozyma,* the green algae such as *Haematococcus pluvialis* and *Paracoccus carotinifaciens,* and the carotenoids concentration of the products thereof are from 0.5 to 5%. In the case of krill, the extracted oil thereof contains about 2000ppm, namely, 0.2%, and further concentration process is necessary for market use. Therefore, the production cost for concentration process increases and it cannot be mentioned to be a competitive raw material.

It has been already reported in 1970s that the concentration of astaxanthin in eyeballs of krill is high (see Non-patent Documents 1 and 2). The present applicant has also reported that the astaxanthin is contained in krill in a high concentration, and particularly, about fourth part of astaxanthin in whole krill is concentrated in eyeballs thereof (see Non-patent Document 3).

There is no conventional art of using the eyeballs of the crustaceans for specific applications, and as shown in Patent Document 1, a specific type of crustaceans is used and eyeballs thereof are removed when they become obstacles for a specific object.
Non-patent Document 1: Shoku no Kagaku (Science of Food), 44, 91-103, 1978
Non-patent Document 2: Shoku no Kagaku (Science of Food), 11, 74-86, 1973
Non-patent Document 3: Nippon Suisan Kaisha, Ltd., Central Research Laboratory, Report No. 11, pp. 18-27, 1976
Patent Document 1: JP-A-2000-166516

### Disclosure of the Invention

### Problems that the Invention is to Solve

The object of the present invention is to provide a method for efficiently utilizing crustaceans, particularly, krill as resources. More particularly, the object of the present invention is to provide a method for efficiently recovering and utilizing lipophilic effective substances such as carotenoids, highly-unsaturated fatty acids, and phospholipids which are components thereof.

### Means for Solving the Problems

An object of the invention is an aggregate of eyeballs of a crustacean, particularly krill or mysid.

Another object of the invention is method of using an aggregate of eyeballs of crustacean, particularly krill or mysid, as a supply source of carotenoids, highly-unsaturated fatty acids and/or phospholipids.

Still another object of the invention is a food or a nutritional supplement for furnishingcarotenoids, highly-unsaturated fatty acids/or phospholipids, said food or said nutritional supplement comprising an aggregate of eyeballs of crustacean, particularly krill or mysid, as it is or in a pulverized state.

Even still another object of the invention is process for producing an aggregate of eyeballs of crustacean, particularly krill or mysid, comprising: reducing a water content of the crustacean; and releasing the eyeballs from fish bodies by physical impact, followed by recovering them. For example, the water contents thereof can be reduced by heating, and after drying is performed such that the water contents thereof are reduced to 15% or less, the eyeballs can efficiently be recovered by using an oscillating sieve.

Even still further another object of the invention is a method for producing a highly-pure aggregate of eyeballs of a crustacean by removing foreign matters other than the eyeballs with a wind grading machine.

### Advantage of the Invention

According to the invention, eyeballs of crustaceans, which are foreign matters when they are merely mixed in whole crustaceans, are converted into useful resources by allowing them into an aggregate of solely eyeballs. This aggregate of the eyeballs can be used as an efficient supply source of carotenoids, highly-unsaturated fatty acids, phospholipids or the like, and can be added to a food, a nutritional supplement or the like as it is or in a pulverized state.

By utilizing a property of the eyeballs such that they are released by reducing the water contents of the crustaceans by the method according to the invention, namely, heating or the like, the eyeballs being small in size can efficiently be recovered.

Further, fish bodies of the crustaceans remaining after the recovery of the eyeballs can be utilized as krill meal or the like as usual, since they have only been subjected to heating.

### Best Mode for Carrying Out the Invention

As for crustaceans to be used in the present invention, any type of crustacean can be used, so long as it contains effective components such as carotenoids, highly-unsaturated fatty acids, phospholipids and the like. The preferable examples thereof include krill or mysid in which contents of carotenoids, particularly, astaxanthin is high. Although other shrimps and crabs can be used, when bodies thereof are large in size, eyeballs thereof are sometimes unduly hard, and in that case, the eyeballs are crushed and then used. Further, since effective components are varied in accordance with types of crustaceans, types of crustaceans are selected in accordance with the purposes.

The term "eyeball of a crustacean" as used herein means a portion connected with the head portion of the crustacean by eye-stalk and is intended to indicate solely an eyeball or eyeball containing eye-stalk.

The term "aggregate of eyeballs" as used herein does not refer to a unit of one, two, or 100 eyeballs but refers to a unit of kg such they are collected to an extent which can industrially be utilized. As for the aggregate of eyeballs, when it is used as a supply source of carotenoids, highly-unsaturated fatty acids and/or phospholipids, those in which the ratio of the eyeballs to the entire weight is concentrated to 20 to 100% can be used. Further, when it is used in a nutritional food or the like as it is, those in which the ratio of the eyeballs is concentrated to from 80 to 100% are preferable. The aggregate in which the ratio of the eyeballs is about 20% can be obtained only by performing drying to facilitate release of the eyeballs, releasing the eyeballs by physical impact, and then recovering the eyeballs. By further subjecting it to wind grading mashine, grading utilizing inclination or the like, an aggregate in which the ratio of the eyeballs is from 20 to 100% can be obtained.

Examples of main effective components contained in the eyeballs of crustaceans include carotenoids, highly-unsaturated fatty acids and phospholipids. Examples of carotenoids include astaxanthin, astaxanthin monoester, and astaxanthin diester; examples of highly-unsaturated fatty acids include eicosapentaenoic acid, docosahexaenoic acid, and linolenic acid; and examples of phospholipids include phosphatidyl choline. Since these components are physiologically active in humans, animals, fish and shellfish or the like or essential nutrients for them, they are utilized in a food, a feed, a supplement or the like.

The aggregate of eyeballs of crustaceans according to the invention can either be used as a food, a feed, or a supplement as it is or be used after being adding thereto in a pulverized form. For example, the eyeballs obtained from heated krill are raw materials as they are such that they only have a taste of dried shrimp, have no strong taste, have a light texture at eating, are fine granular without being crushed and have versatility. Such eyeballs as described above can be added to any foods, so long as the foods are fond of such a shrimp flavor. The eyeballs are also suitable for feeds for fish and shellfish, feeds for livestock, and feeds for growing poultry. Further, they can be utilized as supplements in the form of capsules or tablets.

The aggregate of eyeballs according to the invention can also be used as a supply source from which astaxanthin or the like is extracted. In this case, it can be extracted far more efficiently in comparison with the case in which it is extracted from whole bodies of crustaceans. The eyeballs are crushed and are subsequently subjected to extraction by using extraction solvents and extraction conditions appropriate for products to be extracted. For example, when the eyeballs of krill are subjected to extraction by using hexane, a lipid in which the concentration of astaxanthin is 4% or more can be obtained.

A process for producing an aggregate of eyeballs according to the invention utilizes a property in which reduction of water contents of the crustaceans facilitates release of the eyeballs of crustaceans from other portions. According to the process, the aggregate of eyeballs can be recovered without using any specific apparatus or reagent. This is due to the fact that reduction of the water contents of the crustaceans such as krill or mysid decreases strength of eye-stalks supporting eyeballs and releases eyeballs with weak physical impact.

As for methods of reducing the water content, drying by heating is most efficient. For example, fresh crustaceans are subjected to a primary heating by means of, for example, boiling, steaming, microwave-heating. Joule heating, or induction heating to raise a product temperature to a boiling point of water or less. Although a given temperature is necessary to denaturalize protease inherent in the krill and to suppress decomposition of protein during processing, unduly high temperature may cause deterioration of lipids, or depletion of useful materials such as astaxanthin. By similar reasons, this primary heating is preferably performed in a short period of time. Further, it is necessary to pay attention not to break eyeballs at the time of heating. Subsequently, drying is performed by hot wind or the like. Heating is preferably performed such that a temperature of a subject (eyeballs or the like) does not come to be more than 100°C. Specifically, drying is preferably performed with hot wind of 120°C or less and a net conveyor type dryer or a fluid bed dryer which can continuously perform drying is used. It is necessary to pay attention as much as possible not to break the eyeballs at the time of drying.

As described above, since the eyeballs of the crustaceans subjected to heating and drying can be released with weak physical impact, the eyeballs are released with such a degree of vibration as not break fish bodies of the crustaceans, and then collected by a sieve with an appropriate mesh size.

Drying is performed to such an extent that the water content thereof come to be 15% or less. When drying is not sufficiently performed, yield is deteriorated.

Other drying methods than heating, such as freeze-drying, wind-drying and the like may be used.

Eyeballs, which are released by heating and physical impact and then recovered, contain not only eyeballs but also foreign matters such as antennas and maxillipeds. In order to obtain a highly pure aggregate of eyeballs, it is necessary to remove these foreign matters by wind classification, inclination grading or the like. Since the degree of purity of the aggregate of eyeballs is varied depending on the purposes of applications, it is conducted in accordance with such purposes. Since the foreign matters are fundamentally derived from crustaceans, there are many cases in which the foreign matters do not give any problem depending on applications. When it is required to strictly eliminate the foreign matters, remaining foreign matters are removed by subjecting the aggregate of eyeballs to a color sorter machine or the like.

Since individual krill weight varies widely, as approximate numbers, the total weight of random sampled 100 dried krill was 11 g and then the weight of collected 100 eyeballs of dried krill arbitrarily was 0.1 g. Since one individual krill has two eyeballs, yield of eyeballs of the krill is theoretically about 1.8% of the weight of the krill. When fresh krill is dried, the weight thereof becomes one fifth. Therefore, on calculation, about 3.6 kg of eyeballs of dried krill can theoretically be obtained from one ton of fresh krill.

Hereinafter, examples of the invention are described. However, the invention should not be limited thereto in any way.

### Example 1

### Method

Fresh krill were subjected to a primary heating by steaming to raise a product temperature to a boiling point of water or less (about 90°C), and thereafter, immediately subjected to drying. The drying was performed by using a net conveyor type dryer which can be continuously operated with hot wind drying at 120°C or less. Attention was paid as much as possible at the time of drying not to break the eyeballs. Further, as the drying was proceeded (water content was decreased), the eyeballs came to be easily released from fish bodies of the krill and such release sometimes occurred during drying. These separated eyeballs in process were collected by a cyclone separator installed at dryer exhaust outlet, a moving scraper installed at a lower portion of the net conveyor or the like.

After being subjected to drying, the krill was subjected to a sieve. The sieve used was an oscillating sieve (Type 403, Dalton Co., Ltd.; screen wire diameter: 1.2 mm; opening (square): 6.0 mm). The foreign matters other than eyeballs, which passed through the mesh, were separated with a wind grading machine.

### Results

After drying, approximately 100% of the eyeballs of the krill were released from fish bodies by physical impact. In separated fish bodies, eyeballs such as black spots were not found by a naked eye observation. Matters passed through the mesh were eyeballs and foreign matters such as antennas and maxillipeds. At this stage, a ratio of eyeballs increased to about 20 to 30%. Since the ratio of the eyeballs in the weight of the dried krill is about 1.8%, it can be mentioned that it was concentrated by from about 10 to 15 times.

Since the mixed foreign matters such as antennas and maxillipeds are lighter than eyeballs in weight, they were able to be separated by the wind grading machine. Finally, 16 g of aggregate containing substantially only eyeballs was obtained from 2450 g of dried krill. Since the ratio of the eyeballs in the dried whole krill is theoretically about 1.8%, the theoretical yield of the eyeballs was 36%.

By examining conditions and adjusting functions of the wind grading machine such that the mesh size of the sieve was set to be from 2.5 to 3.0 mm through which eyeballs just passed, the aggregate containing substantially only eyeballs was able to be recovered with a recovery rate of about 50%. Further, by the separation with an inclined conveyor-belt machinery (an apparatus for separating a spherical body (eyeball) from non-spherical body by transporting on an inclined conveyor-belt machinery), an aggregate containing substantially only eyeballs was able to be recovered with a theoretical yield of 80% or more.

The obtained aggregate of krill eyeballs was subjected to chemical component analysis. The results are shown below.
water content: 1.5% (measured by infrared moisture meter)
lipid content: 8.5% (measured by Brigh-Dyer method)
total carotenoid: 2990 ppm (After krill eyeballs are dissolved in acetone, absorbance at 478 nm was measured.)
lipid composition: shown in Table 1 (Lipid classes were measured by using TLC-FLD.)
fatty acid composition: shown in Table 2 (Total lipid was methylated by BF₃ methanol method, and then measured by GC.)

**Table 1**

| Components | Content (%) |
|---|---|
| Wax | 2.2±0.5 |
| Triacylglycerol | 15.4±0.9 |
| Free fatty acid | 1.7±0.2 |
| Sterol | 5.8±0.3 |
| Phospholipid | 74.9±1.1 |

**Table 2**

| FA species | Content (%) |
|---|---|
| C14:0 | 5.6 |
| C16:0 | 19.2 |
| C16:1n-7 | 2.1 |
| C16:2n-4 | 1.5 |
| C18:0 | 1.6 |
| C18:1n-9 | 6.3 |
| C18:1n-7 | 5.7 |
| C18:2n-6 | 1.7 |
| C18:3n-3 | 3.4 |
| C18:4n-3 | 4.5 |
| C20:4n-6 | 0.6 |
| C20:4n-3 | 0.5 |
| C20:5n-3 | 18.4 |
| C22:5n-3 | 0.4 |
| C22:6n-3 | 22.3 |
| Others | 6.2 |

### Example 2

A quick frozen boiled krill on a boat was thawed, heated and dried, and then eyeballs of the krill and a fish body without eyeballs were obtained by a method according to the present invention and were subjected to astaxanthin extraction. A freeze-dried boiled krill was used as reference.

600 mg of each of crushed samples was weighed and was extracted three times by using 50 times its volume of hexane, and then a hexane-soluble lipids containing astaxanthin was obtained. After thus-obtained lipids were concentrated and weighted. Subsequently, it was dissolved in acetone, and then absorbance at 478 nm was measured to determine astaxanthin concentration.

As shown in Table 3, the concentration of astaxanthin in the lipids of the eyeballs of krill was considerably high in comparison with that of the whole body, and only by performing a hexane extraction, the lipids in which the astaxanthin concentration was about 4% was able to be obtained. When astaxanthin is extracted from krill, by extracting astaxanthin not from the whole body but from collected eyeballs, a lipid containing astaxanthin in a high concentration can efficiently be obtained by a small amount of solvent.

The reason why the content of astaxanthin in the heated and dried product of the krill is lower than that of the freeze-dried product of the krill as a reference, is that astaxanthin is possibly depleted by heating. In this Example, in order to sufficiently perform drying, heating-drying was performed for 7 hours at 80°C. In this regard, it is preferable to select a heating temperature and a heating period of time allowing such depletion of astaxanthin to be minimal. However, even when this depletion is taken into consideration, it is confirmed that the method for extracting astaxanthin in accordance with the method of the invention is sufficiently useful.

**Table 3**

| | Lipid content (%) | Astaxanthin content (ppm) | Astaxanthin concentration in lipid (%) |
|---|---|---|---|
| Eyeballs of krill | 4.61 | 1863 | 4.04 |
| Fish body of krill (eyeballs removed) | 12.19 | 37 | 0.03 |
| Heated and dried product of krill* (whole body of krill) | 12.05 | 70 | 0.06 |
| Freeze-dried product of krill (whole body of krill) | 12.57 | 129 | 0.10 |

| | | | |
|---|---|---|---|
| (* : Data of heated and dried product of krill is a calculated value from data of eyeballs and fish body.) | | | |

### Example 3

Four parts of microcrystalline cellulose and one part of magnesium stearate were mixed to one part of the lipids containing astaxanthin extracted from the eyeballs according to Example 2. Then, the resultant mixture was subjected to tablet-making, to thereby produce tablets.

### Example 4

To the fat containing astaxanthin extracted from the eyeballs according to Example 2, 1% each of vitamin C and vitamin E were added. Then, the resultant mixture was filled in dark-brown gelatin-made soft capsules in accordance with an ordinary method, to thereby produce capsules.

### Example 5

Five percent of lipids containing astaxanthin extracted from the eyeballs according to Example 2, 9% of microcrystalline cellulose, 75% of sucrose, 1% of magnesium stearate and 10% of ascorbic acid were mixed with one another. Then, tablets were produced from the resultant mixture by an ordinary method.

### Industrial Applicability

An aggregate of eyeballs of a crustacean according to the present invention can be used as a supply source of effective components such as a carotenoid, highly-unsaturated fatty acids or phospholipids. It can be used to a food, feed, nutritional supplement and the like as it is or in a pulverized state, and it can also be used as a raw material for extracting the carotenoid, the highly-unsaturated fatty acids, the phospholipids or the like. The eyeballs of the crustacean, which have conventionally been treated as rather obstacles and impulities, can be utilized in a useful manner.

## Claims

1. An aggregate of eyeballs of a crustacean.

2. The aggregate of eyeballs according to claim 1, wherein the crustacean is krill or mysid.

3. A method of using an aggregate of eyeballs of a crustacean as a supply source of carotenoids, highly-unsaturated fatty acids and/or phospholipids.

4. The method of using an aggregate of eyeballs of a crustacean according to claim 3, wherein the aggregate of eyeballs of a crustacean is subjected to a solvent extraction to thereby obtain a carotenoid-containing lipid.

5. The method of using an aggregate of eyeballs of a crustacean according to claim 3 or 4, wherein the crustacean is krill or mysid.

6. A food or a nutritional supplement for furnishing carotenoids, highly-unsaturated fatty acids/or phospholipids, said food or said nutritional supplement comprising an aggregate of eyeballs of a crustacean as it is or in a pulverized state.

7. The food or the nutritional supplement according to claim 6, wherein the crustacean is krill or mysid.

8. A process for producing an aggregate of eyeballs of a crustacean comprising:
reducing a water content of the crustacean; and
releasing the eyeballs from a fish body by physical impact, followed by recovering them.

9. The process for producing an aggregate of eyeballs of a crustacean according to claim 8, wherein the water content is reduced by heating.

10. A process for producing an aggregate of eyeballs of a crustacean comprising:
drying the crustacean by reducing a water content thereof to 15% or less by heating; and
subjecting the crustacean to vibrate on a sieve to thereby recover the eyeballs.

11. A process for producing a highly-pure aggregate of eyeballs of a crustacean comprising:
drying the crustacean by reducing a water content thereof to 15% or less by heating;
subjecting the crustacean to vibrate on a sieve, followed by recover the eyeballs; and
removing a foreign matter other than the eyeballs with a wind grading machine or an inclined grading machine.

12. The process for producing a highly-pure aggregate of eyeballs of a crustacean according to any one of claims 8 to 11, wherein the crustacean is krill or mysid.
